# EUROPEAN PATENT APPLICATION

(11) **EP 1 935 364 A1**
(43) Date of publication of application: **25.06.2008**
(21) Application number: 07255008.0
(22) Date of filing: 21.12.2007
(51) Int. Cl.: A61B 19/00, A61L 2/18, A61L 2/24

(54) **Instrument soaking container and method**

(30) Priority: 22.12.2006 US 615279
(71) Applicant: ETHICON, INC., Somerville, New Jersey 08876 (US)
(72) Inventor: Lin, Szu-Min, Irvine, CA 92620 (US); Platt, Robert C., Laguna Niguel, CA 92677 (US); Zhu, Peter C, Cupertino, CA 95014 (US); Raja, Vishnu R, Irvine, CA 92614 (US)
(74) Representative: Tunstall, Christopher Stephen

(57) **Abstract**

A container (10) provides for soaking medical instruments (12). The container (10) includes a basin (14) for receiving one or more medical instruments (12), a fill level sensor (24) for detecting presence of a soaking substance (18) at a minimum fill level (20), a lid closure sensor (26) which detects closure of a lid (16) onto the basin (14), and a timer adapted to begin a timing sequence when both the fill lever (24) sensor detects presence of the soaking substance (18) at the minimum fill level (20) and the lid closure sensor (26) detects closure of the lid (16). Proper soaking of the instruments (12) prior to a full cleaning procedure improves the reliability of the cleaning procedure.

## Description

### Field of the Invention

This application relates to preparing medical instruments for reuse after a medical procedure, and more specifically to insuring that the instruments receive a sufficient amount of time in a soaking solution after their medical use.

### Background of the Invention

Many medical instruments are designed to be reused after a particular procedure. However, after having come into contact with bodily fluids and material, deposits can form on these instruments, which are difficult to remove in a clean procedure. It is recommended that instruments be soaked for a time period prior to their being cleaned and sterilized.

Insuring that a sufficient soaking period is achieved while achieving rapid turnaround in the time from use of the instrument in a surgical procedure until it is ready for reuse can be a challenge. In some instances, the personnel who put the instruments into a container for soaking differ from the personnel who may remove the instrument from that container, and the removal may occur in a physically distant location in the medical setting. For instance, nurses in an operating room may put the instruments in for their initial soaking, and later personnel in the central supply room of the hospital where the subsequent cleaning and sterilization is to occur may remove them from the soaking solution. Effective communication is required to insure that a sufficient time for soaking has occurred without delaying processing of the instruments after such sufficient time has actually occurred. It is also important to make sure that a sufficient depth of soaking solution is achieved in the container so that all portions of the instrument remain submerged.

### Summary of the Invention

The present invention overcomes these and other limitations in the prior art.

A container, according to the present invention, provides for soaking medical instruments. The container includes a basin for receiving one or more medical instruments, a fill level sensor for detecting presence of a soaking substance at a minimum fill level, a lid closure sensor which detects closure of a lid onto the basin, and a timer adapted to begin a timing sequence when both the fill lever sensor detects presence of the soaking substance at the minimum fill level and the lid closure sensor detects closure of the lid. Proper soaking of the instruments prior to a full cleaning procedure improves the reliability of the cleaning procedure.

Preferably, the container incorporates a screen at or below the minimum fill level to enforce submersion of buoyant instruments. Conveniently, this would attach to the lid.

Preferably, instructions are included for loading one or more instruments into the container, filling the container to at least the minimum fill level, closing the lid and allowing the instruments to soak for a time period timed by the timer.

Preferably, an indicator on the container indicates a successful soaking cycle after the timer has run for a predetermined time period.

Preferably, a communication system is provided for communicating data between the container and an external monitoring or control system, which could be either local, such as a handheld device used at the container or remote, such as a host computer located in a different part of the building, or even perhaps accessed over the internet at a location geographically removed from where the container is located.

Preferably, the soaking substance incorporates a cleaning fluid, a disinfectant or a sterilant. Hydrogen peroxide is preferred for its ability to dissolve dried blood and to quickly inactivate common pathogens of concern to hospital personnel. Preferably it is present in the soaking substance from 3% to 10% by weight, and more preferably from 4% to 6% by weight.

A method, according to the present invention, provides for ensuring proper soaking of instruments. The method comprising the steps of: a) placing one or more instruments into a basin of a container; b) filling the container to a minimum fill level with a soaking substance and detecting such via a minimum fill sensor; c) closing the container with a lid and detecting such via a lid closure sensor; and d) upon completion of both steps c) and d), initiating a timer cycle to time soaking of the instruments.

Preferably, successful completion of a soaking cycle comprises the lid remaining closed and the level of soaking substance remaining at or above the minimum fill line during a predetermined time, and the method further includes the step of displaying the successful completion of the soaking cycle to the user. The time preferably exceeds 5 minutes.

In one aspect of the invention the soaking substance comprises a foam.

### Brief Description of the Drawings

FIG. 1 is a front elevation view of a container of the present invention;
FIG. 2 is a block diagram of a communication system for use with the container of FIG. 1; and
FIG. 3 is a block diagram of a handheld device for use with the container of FIG. 1 and communication system of FIG 2.

### Detailed Description of the Invention

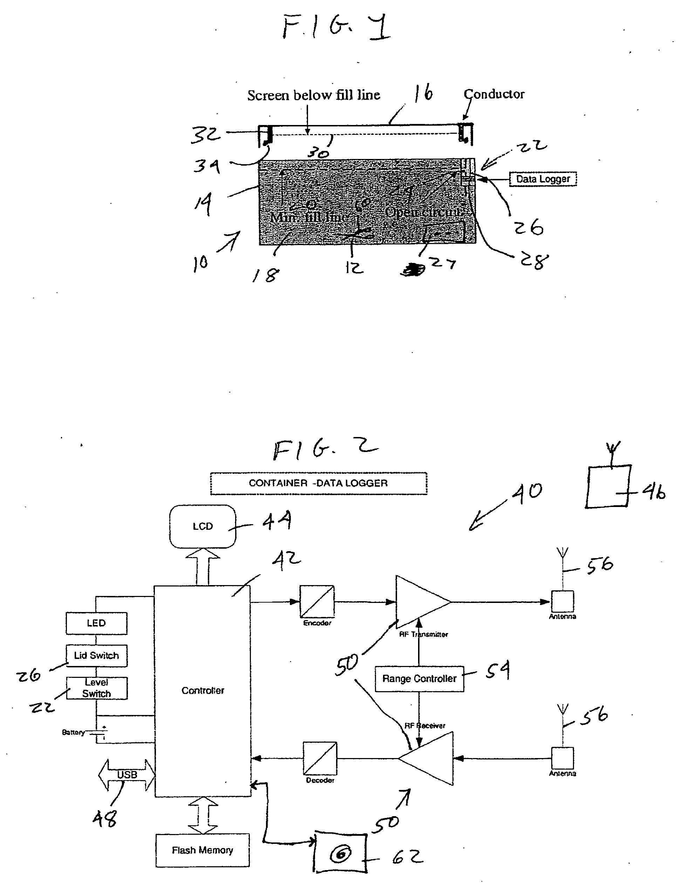

FIG. 1 discloses a container 10 adapted for soaking medical instruments 12 after their use in a medical procedure. It comprises a basin 14 and a lid 16 which fits upon the basin 14. The basin 14 is preferably fluid tight and sized to accommodate one or more of the surgical instruments 12 and to accommodate a soaking solution 18 up to a fill line 20. Preferably, the lid 16 is also fluid tight and tightly fitting such that when the lid 16 is placed upon the basin 14 the container 10 becomes fluid tight such that the solution 18 cannot easily spill out.

A soaking indicator system 22 on the container 10 aids in determining whether sufficient soaking time has been provided to the instruments 12. This system 22 comprises in gross a fill sensor 24 at the fill line 20 to detect the proper depth of solution 18, a lid closure sensor 26 to detect proper closure of the lid 16, and a data logger 28 having a timing function to time the lid 16 being closed with solution 18 to the fill line 20. In simple form, the soaking indicator system 22 employs a moisture sensor for the fill sensor 24, a contact switch for the lid closure sensor 26 and a simple countdown timer for the data logger which initiates its timing upon activation of the fill sensor 24 and lid closure sensor 26 and then provides an indication at the end of a predetermined soaking time such as by lighting an LED 27. For instance a red LED could indicate that the cycle is not yet complete and a greed LED could indicate that the cycle is complete, preferably with labels for each LED. Of course, more sophisticated systems may also be employed.

One important feature of the lid 16 is a screen 30 which is disposed below the lid 16 and sits at or below the fill line 20 when the lid 16 is closed. The screen 30 insures that buoyant instruments 12 will not float up above the level of the solution 18 and have certain portions of themselves avoid proper soaking. Preferably, it is supported on standoffs 32. For economy and construction, one of the standoffs 32 can be adapted to engage the lid closure sensor 26. Further, the standoffs 32 may bear a seal 34, as for instance silicone, which bears against the container 16 and helps to maintain leaktight configuration when the lid 16 is closed. The seal 34 could be located on other locations of the lid 16 or where the lid 16 contacts the basin 14. One or more latches, not shown, may be provided for holding the lid 16 closed.

A more sophisticated data logger 40 is shown in FIG. 2. The data logger 40 mounts on the wall of the soaking container 10 and incorporates the fill sensor 22 and the lid closure sensor 26 each of which are connected in series to a controller 42.

The controller 42 receives a high input signal only when both the lid closure 26 and liquid fill 22 sensors are closed. A high input signal to the controller 42 starts a soaking timer within the controller 42. Preferably the status of the sensors 22 and 26 and other information such as the soaking duration are displayed on a display 44, such as an LED or LCD display. The display 44 can also include a count down timer output showing the time remaining until a full soaking has been completed. Preferably it further provides some indication at to whether there is enough liquid and whether the lid is closed properly. During soaking time if either the lid is opened or the liquid level falls below the minimum fill line, the timer will reset. Satisfactory completion of a soaking cycle is displayed on the display 44.

Further, information regarding the soaking cycle is transferred to a remote base station host 46 for further processing. Such information can include the soaking time, time of completion etc. Transfer of the information to the host 46 can occur in a multitude of ways, such as through a USB link 48, RF transceiver 50, RFID (not shown) or manual entry.

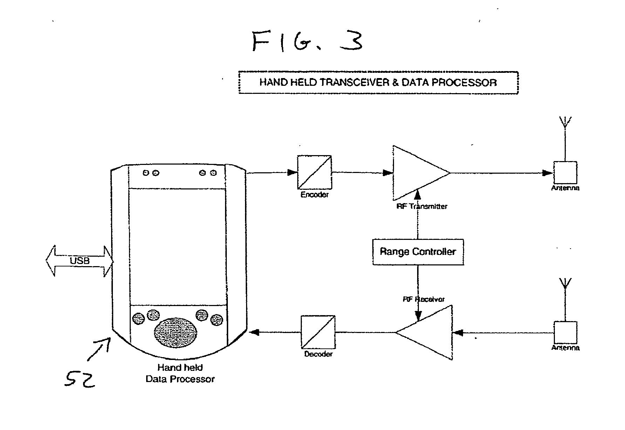

If using the RF transceiver 50 to communicate with base station and an optional hand held device 52 (FIG. 3), communication is controlled through a range controller 54 by adjusting communication frequency and/or power. The data logger controller 42 encodes and transmits data through its RF transceiver 50 using an antenna 56. Similar equipment at the base station 46 and handheld device 52 receive the signal from the antenna 56 and decode the data. Preferably, the data logger communicates with the base station 46 and hand held device 52 through high frequency and low frequency RF, respectively and the base station 46 communicates with the hand held device 52 through low frequency RF communication.

The data transferred can include a unique container identifier, soak time, user information, instrument list, fluid level, fluid type, lid status, record of container usage, instruments, record of instrument usage, and record of instrument processing methods including cleaning, disinfection or sterilization. The information can be communicated visually or electronically, locally or remotely. By viewing or retrieving the information, user can know whether the container has enough fluid, what type of fluid is in the container, whether the lid is closed properly, whether the instrument has been soaked long enough, the owner of the container, the history of the container, instrument in the container, the number of instruments, the history of the instrument, and the next processing step after soaking. The next step can be for further cleaning, decontamination, disinfection, or sterilization. The decontamination, disinfection or sterilization can be either low temperature or higher temperature process. It can also be a specific washer, decontaminator, or washer/decontaminator.

When the amount of information is small the display 44 should suffice. When more copious amounts of information are handled the handheld device 52 is preferred. It ideally communicates directly with the container's transceiver 50. Existing communication protocols such as Bluetooth or WiFi are preferred, but the invention need not be so limited.

Information about the instruments 12, such as the type, number and ID numbers thereof can be manually entered, either on a keypad or entry device on the container 10, but more preferably through the handheld device 52. A more convenient method would be to tag each instrument 12 with a machine readable tag, such as an RFID tag 60 (FIG. 1). Then, an RFID tag reader 62 incorporated into the data logger 40, or in the handheld device 52 could read and record the information automatically. Therefore, the container 10 can communicate with the user whether all previously removed instruments are properly back to in the container 10. Preferably, the display 44, or the handheld device 52, will indicate when all of the instruments 12 are in the container 10, and if not which ones are missing. The user can also track the location of the container 10, the use of the container 10, and the use of instruments 12 in the container. The information can be delivered to the remote host 46 for processing and storage. Optionally, the host 46 can receive container information and then send necessary information to the handheld device 52 to notify user of the container status or information.

Depending upon the types of instrument 12 or perhaps even the type of procedure in which it was used, the length of time for soaking can be modified. The type or strength of soaking solution may also be adapted. Preferably, this is automatically determined by the onboard controller 42, or by the handheld device 52 or the remote host 46 and then communicated to the onboard controller 42. If a change in soaking fluid is involved it is preferably communicated to a user by being displayed on the display 44 or on the handheld device 52.

In use, one or more instruments 12 are placed into the basin 14. Typically they are placed in the basin 14 as their use in a surgical or other medical procedure is completed. The soaking solution 18 is then placed over the instruments. The solution 18 may not be topped up to the minimal fill line 20 until all the instruments 12 are in the basin 14. When the solution 18 reaches the minimum fill line 20 the fill sensor 24 detects this and closes. After all the instruments 12 are in the basin 14 and sufficient solution 18 has been added to reach the minimum fill line 20, then the lid 16 is closed. Closure of the lid closes the lid closure sensor 26. When both sensors 24 and 26 have closed the data logger timer begins to run. After running for a predetermined time an indication of completion is provided such as by lighting the LED 27.

Different soaking solutions 18 are appropriate for use with the present invention. It may be a cleaning fluid, a disinfectant, or a sterilant. Preferably, the fluid has combined features of cleaning/disinfection or cleaning/sterilization. One well known cleaning solution is ENZOL enzymatic soaking solution available from Advanced Sterilization Products division of Ethicon, Inc. located in Irvine, CA. Alternatively, a solution of hydrogen peroxide (preferably 3 to 10% by weight, more preferably 4 to 6%) can be employed. A further option would be so employ a foam, such as a foam comprised of hydrogen peroxide, preferably incorporating a corrosion inhibitor and a lipid dissolving agent. A foam has the advantage of having less mass easing handling of the filled container and reducing the chances of spillage. A suitable foam is disclosed in co-pending U.S. Application Serial No. 11/565,126, filed November 30, 2006, the contents of which are incorporated herein by reference.

The minimum soaking time depends upon the goal sought. For soaking in the 6% peroxide foam five minutes is sufficient to dissolve dried blood, and for a 3% foam ten minutes. Such soaking times are also sufficient to inactivate most common pathogens of concern to hospital personnel.

The foam volume may decrease over time. Therefore, container with foam may have an automated triggering mechanism to regenerate foam in the container to the required level. The mechanism may be an agitator on the slopped bottom of the container. The agitator may be driven by a motor and a power source. Alternatively, air can be pumped through the foam. The triggering mechanism may be a timer or a fluid level sensor.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A container for soaking medical instruments, the container comprising:
a basin for receiving one or more medical instruments;
a fill level sensor for detecting presence of a soaking substance at a minimum fill level;
a lid closure sensor which detects closure of a lid onto the basin; and
a timer adapted to begin a timing sequence when both the fill lever sensor detects presence of the soaking substance at the minimum fill level and the lid closure sensor detects closure of the lid.

2. A container according to claim 1 and further comprising a screen at or below the minimum fill level wherein to enforce submersion of buoyant instruments.

3. A container according to claim 1 and further comprising instructions for loading one or more instruments into the container, filling the container to at least the minimum fill level, closing the lid and allowing the instruments to soak for a time period timed by the timer.

4. A container according to claim 1 and further comprising an indicator indicating a successful soaking cycle after the timer has run for a predetermined time period.

5. A container according to claim 1 and further comprising a communication system for communicating data between the container and an external monitoring or control system.

6. A container according to claim 5 wherein the external monitoring or control system is remotely located from the container.

7. A container according to claim 1 wherein the soaking substance comprises at least one of a cleaning fluid, a disinfectant or a sterilant.

8. A container according to claim 8 wherein the soaking substance comprises hydrogen peroxide.

9. A container according to claim 8 wherein the hydrogen peroxide is present in the soaking substance from 3% to 10% by weight.

10. A container according to claim 9 wherein the hydrogen peroxide is present in the soaking substance from 4% to 6% by weight.

11. A method for ensuring proper soaking of instruments, the method comprising the steps of:
a) placing one or more instruments into a basin of a container;
b) filling the container to a minimum fill level with a soaking substance and detecting such via a minimum fill sensor;
c) closing the container with a lid and detecting such via a lid closure sensor; and
d) upon completion of both steps c) and d), initiating a timer cycle to time soaking of the instruments.

12. A method according to claim 11 and further comprising the step of submerging a buoyant instrument by holding it submerged with a screen at or below the minimum fill level.

13. A method according to claim 11 wherein a successful completion of a soaking cycle comprises the lid remaining closed and the level of soaking substance remaining at or above the minimum fill line during a predetermined time, the method further comprising the step of displaying the successful completion of the soaking cycle to the user.

14. A method according to claim 11 wherein the soaking substance comprises hydrogen peroxide.

15. A method according to claim 11 wherein the soaking substance comprises a foam.

16. A method according to claim 11 wherein successful completion of a soaking cycle comprises the lid remaining closed and the level of soaking substance remaining at or above the minimum fill line during a predetermined time, and wherein the predetermined time exceeds 5 minutes.

17. A method according to claim 11 and further comprising the step of transferring data regarding the soaking cycle to an external monitoring or control system.
